# EUROPEAN PATENT APPLICATION

(11) **EP 2 455 129 A1**
(43) Date of publication of application: **23.05.2012**
(21) Application number: 11189379.8
(22) Date of filing: 16.11.2011
(51) Int. Cl.: A61M 25/00

(54) **Guide catheter composed of shape memory polymer**

(30) Priority: 17.11.2010 US 414577 P
(71) Applicant: Micrus Endovascular LLC, San Jose, California 95131 (US)
(72) Inventor: Williams, Eric, Miramar, FL 33026 (US)
(74) Representative: Small, Gary James

(57) **Abstract**

A guide catheter (10) includes a tubular section (24) formed of a shape memory polymer that can transform dynamically between first (32) and second (34) states or conditions, or among three states or conditions, to provide a wide range of properties of the guide catheter as desired during delivery of the guide catheter through the vasculature to a target site, and removal of the guide catheter from the target site and vasculature. The states or conditions of the shape memory polymer be dynamically changed to vary properties of the tubular section such as stiffness, flexibility, shape, or biodegradability, which can be controlled by exposure of the shape memory polymer to temperature changes, electric fields, magnetic fields, wavelengths of light, and chemical solutions.

## Description

### BACKGROUND OF THE INVENTION

The present invention relates generally to guiding catheters, and more particularly relates to the use of a shape memory polymer for controlling the stiffness, flexibility or shape of at least a portion of a guiding catheter.

Guiding catheters are catheters that are utilized in various medical procedures, including venography and implanting of neuro or cardiac devices for locating and cannulating vessels in a patient's vasculature, such as in a patient's heart or carotid arteries, for example. Cannulating small convoluted vessels requires navigating a small diameter, flexible guide through the vasculature into the destination vessel. Once the destination vessel is reached, the guiding catheter typically acts as a conduit for insertion of a therapeutic device into the vessel. A pre-shaped guiding catheter is typically used to blindly locate the vertebral or carotid artery. This endeavor, however, is complicated by the fact that the location of the vertebral or carotid may vary appreciably from one patient to another, especially among patients with vascular disease, and physicians frequently need to gain further access into the vasculature.

A fixed shaped catheter is adequate in many cases where the pathway is not significantly convoluted and the pathway does not deviate significantly between patients. In situations where structural anomalies or significant variations exist, use of a fixed shape catheter may require that the clinician stock multiple sizes and shapes of catheters to account for potential variations. Fixed shaped catheters can require a time consuming trial and error process of inserting and removing different shapes until the destination vessel is successfully accessed.

There is a need for an improved guide catheter for accessing vessels, such as small or convoluted vessels in the neurovasculature, for example, that can dynamically account for anatomical variations and defects associated with destination structures, and that can transform dynamically between a first state or condition to a second state or condition as needed, for delivery through the vasculature, to support treatment at a target site, and for removal from the target site and vasculature. There is also a need for an improved guide catheter for accessing vessels in the neurovasculature that can transform dynamically among first, second and third states or conditions as needed, for delivery through the vasculature, to support treatment at a target site, and for removal from the target site and vasculature. The present invention meets these and other needs.

### SUMMARY OF THE INVENTION

Briefly and in general terms, the present invention provides for a guide catheter including a tubular section formed of a shape memory polymer that can transform dynamically between first and second states or conditions, or among any of three states or conditions, to provide a wide range of properties of the guide catheter as desired during delivery of the guide catheter through the vasculature to a target site, and removal of the guide catheter from the target site and vasculature.

The present invention accordingly provides for a guide catheter including a proximal tubular main body having a proximal portion and a distal portion, a distal tubular section having a proximal portion and a distal portion, and an intermediate tubular section having a proximal portion and a distal portion. The intermediate tubular section is connected between the proximal tubular main body and the intermediate tubular section, and a common lumen interconnects and extends through the proximal tubular main body, the intermediate tubular section and the distal tubular section. The intermediate tubular section advantageously is formed of a shape memory polymer having at least a first state or condition having a first set of one or more properties, and a second state or condition having a corresponding second set of one or more properties, at least one of which is different from the first set of one or more properties.

In a presently preferred aspect, the first state or condition includes a first degree of stiffness of the intermediate tubular section, the second state or condition comprises a second degree of stiffness of the intermediate tubular section, and the second degree of stiffness of the intermediate tubular section is greater than the first degree of stiffness of the intermediate tubular section. In another presently preferred aspect, the first state or condition includes a first degree of flexibility of the intermediate tubular section, the second state or condition includes a second degree of flexibility of the intermediate tubular section, and the first degree of flexibility of the intermediate tubular section is greater than the second degree of flexibility of the intermediate tubular section. In another presently preferred aspect, the first state or condition includes a first shape of the intermediate tubular section, the second state or condition includes a second shape different from the first shape of the intermediate tubular section. The first shape can be a curved shape providing a predetermined angular configuration of the intermediate tubular section, while the second shape can be substantially straight, for example. In another presently preferred aspect, the intermediate tubular section is relatively more biodegradable in the second state or condition than in the first state or condition.

In another presently preferred aspect, transition of the shape memory polymer between the first state or condition to the second state or condition is induced by exposure of the shape memory polymer to a temperature change, such that the shape memory polymer is in the first state or condition when subjected to a first predetermined temperature range, and the shape memory polymer is in the second state or condition when subjected to a second predetermined temperature range. Alternatively, a transition of the shape memory polymer between the first state or condition to the second state or condition can be induced by exposure of the shape memory polymer to one or more predetermined temperature ranges, exposure of the shape memory polymer to one or more predetermined electric fields, exposure of the shape memory polymer to one or more predetermined magnetic fields, exposure of the shape memory polymer to one or more predetermined wavelengths of light, exposure of the shape memory polymer to one or more predetermined chemical solutions, or combinations thereof.

In another presently preferred aspect, the shape memory polymer has a third state or condition, such as a third shape different from the first and second shapes of the intermediate tubular section, although the third state or condition may also include a set of one or more properties selected from stiffness, flexibility, shape and biodegradability, and combinations thereof, corresponding to the first and second sets of properties of the first and second states or conditions. Transition of the shape memory polymer among the first, second and third states or conditions can be induced by exposure of the shape memory polymer to a plurality of predetermined temperature ranges, exposure of the shape memory polymer to a plurality of predetermined electric fields, exposure of the shape memory polymer to a plurality of predetermined magnetic fields, exposure of the shape memory polymer to a plurality of predetermined wavelengths of light, exposure of the shape memory polymer to a plurality of predetermined chemical solutions, or combinations thereof.

Other features and advantages of the present invention will become more apparent from the following detailed description of the preferred embodiments in conjunction with the accompanying drawings, which illustrate, by way of example, the operation of the invention.

### BRIEF DESCRIPTION OF THE WINGS

Figure 1 is a perspective view of a guide catheter according to the present invention, illustrating a first state or condition of the intermediate tubular section.

Fig. 2 is a perspective view of the guide catheter of Fig. 1, illustrating a second state or condition of the intermediate tubular section.

### DETAILED DESCRIPTION OF THE PREFERRED EMBODIMENTS

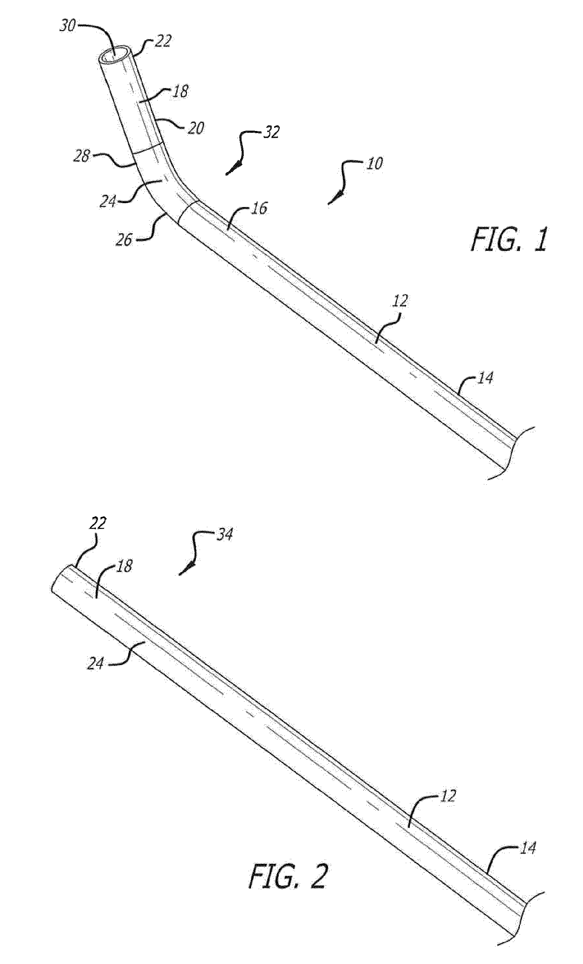

Referring to the drawings, which are provided by way of example, and not by way of limitation, the present invention provides for a guide catheter 10, including a proximal tubular main body 12 having a proximal portion 14 and a distal portion 16, a distal tubular section 18 having a proximal portion 20 and a distal portion 22, and an intermediate tubular section 24 having a proximal portion 26 and a distal portion 28. The proximal portion of the intermediate tubular section is connected to the distal portion of the proximal tubular main body, the distal portion of the intermediate tubular section is connected to the proximal portion of the distal tubular section, and a common lumen 30 interconnects and extends through the proximal tubular main body, intermediate tubular section and the distal tubular section. In a presently preferred aspect the intermediate tubular section is advantageously formed of a shape memory polymer having a first state or condition 32, such as a curved shape or configuration providing a predetermined angular configuration of the intermediate tubular section, for example, illustrated in Fig. 1, and a second state or condition 34, such as a substantially straightened configuration of the intermediate tubular section, for example, illustrated in Fig. 2.

Most shape memory polymers can retain two shapes, and the transition between those is induced by temperature. In some recent shape memory polymers, heating to certain transition temperatures allows a fix of up to three different shapes. In addition to temperature change, the shape change of shape memory polymers can also be triggered by an electric or magnetic field, light or solution. Shape memory polymers can also have a wide variety of other properties that can change between first and second different states or conditions, or among three different states or conditions, such as from stable to biodegradable, from soft to hard, from elastic to rigid, and the like depending on the structural units that constitute the shape memory polymers. Shape memory polymers that can be used in the present invention include thermoplastic and thermoset (covalently crosslinked) polymeric materials.

In general the dynamic transition of properties and/or shapes of the guide catheter of the present invention provides the guide catheter with the ability to change useful characteristics of the guide catheter, such as from a very soft device for accessing very distal vessel to a stiffer state for support while at the target site and return to a softer state for removal of the device, for example.

The first and second states or conditions can encompass one or more properties, such as first and second degrees of stiffness, flexibility, biodegradability, or shapes, for example, although other similar variance of properties of the intermediate tubular section may also be suitable. Typically, when the first and second states or conditions include first and second degrees of stiffness, the second degree of stiffness of the intermediate tubular section is greater than the first degree of stiffness of the intermediate tubular section. Similarly, when the first and second states or conditions include first and second degrees of flexibility, the first degree of flexibility of the intermediate tubular section is greater than the second degree of flexibility of the intermediate tubular section. The intermediate tubular section can also be made to be relatively non-biodegradable or stable in the first state or condition, and relatively more biodegradable in the second state or condition than in the first state or condition. Similarly, when the first and second states or conditions include first and second shapes, the first shape can be a curved shape providing a predetermined angular configuration of the intermediate tubular section, while the second shape can be substantially straight, for example, as illustrated in Figs. 1 and 2.

A transition of the shape memory polymer between the first state or condition to the second state or condition can, for example, be induced by exposure of the shape memory polymer to changes in temperature, changes in an electric field or magnetic field, exposure to different wavelengths of light, chemical solutions, or combinations thereof, or other similar dynamic, controllable environments for the shape memory polymer that can affect the properties of the shape memory polymer. For example, when the transition between the first state or condition and second state or condition can be induced by exposure of the shape memory polymer to changes in temperature, the shape memory polymer typically is in the first state or condition when subjected to a first predetermined temperature range, and the shape memory polymer is in the second state or condition when subjected to a second predetermined temperature range different from the first temperature range. Similarly, when a transition of the shape memory polymer between the first state or condition to the second state or condition can be induced by exposure of the shape memory polymer to a predetermined electric field, the shape memory polymer typically is in the first state or condition when subjected to a first predetermined electric field, and the shape memory polymer is in the second state or condition when subjected to a second predetermined electric field different from the first predetermined electric field. Similarly, when a transition of the shape memory polymer between the first state or condition to the second state or condition can be induced by exposure of the shape memory polymer a predetermined magnetic field, the shape memory polymer typically is in the first state or condition when subjected to a first predetermined magnetic field, and the shape memory polymer is in the second state or condition when subjected to a second predetermined magnetic field different from the first predetermined magnetic field.

Similarly, when a transition of the shape memory polymer between the first state or condition to the second state or condition can be induced by exposure of the shape memory polymer to a predetermined wavelength of light, the shape memory polymer typically is in the first state or condition when subjected to a first wavelength of light, and the shape memory polymer is in the second state or condition when subjected to a second predetermined wavelength of light different from the first predetermined wavelength of light.

Similarly, when a transition of the shape memory polymer between the first state or condition to the second state or condition can be induced by exposure of the shape memory polymer to a predetermined chemical solution, the shape memory polymer typically is in the first state or condition when subjected to a first predetermined chemical solution, and the shape memory polymer is in the second state or condition when subjected to a second predetermined chemical solution different from the first predetermined chemical solution.

The shape memory polymer can also have a third state or state or condition which can encompass a third set of one or more properties corresponding to the first and second sets of properties, such as first and second degrees of stiffness, flexibility, biodegradability, or shapes, such as a third shape different from first and second shapes of the intermediate tubular section, for example, although other similar variance of properties of the intermediate tubular section may also be suitable. Transition of the shape memory polymer among the first, second and third states or conditions can induced by exposure of the shape memory polymer to a temperature change, wherein the shape memory polymer is in the third state or condition when subjected to a third predetermined temperature range, for example. Alternatively, transition of the shape memory polymer among the first, second and third states or conditions can be induced by exposure of the shape memory polymer to a plurality of predetermined temperature ranges, exposure of the shape memory polymer to a plurality of predetermined electric fields, exposure of the shape memory polymer to a plurality of predetermined magnetic fields, exposure of the shape memory polymer to a plurality of predetermined wavelengths of light, exposure of the shape memory polymer to a plurality of predetermined chemical solutions, or combinations thereof.

It will be apparent from the foregoing that while particular forms of the invention have been illustrated and described, various modifications can be made without departing from the spirit and scope of the invention. Accordingly, it is not intended that the invention be limited, except as by the appended claims.

## Claims

1. A guide catheter, comprising:
a proximal tubular main body having a proximal portion and a distal portion and a lumen therethrough;
a distal tubular section having a proximal portion and a distal portion and a lumen therethrough; and
an intermediate tubular section having a proximal portion and a distal portion and a lumen therethrough, said proximal portion of said intermediate tubular section connected to said distal portion of said proximal tubular main body, said distal portion of said intermediate tubular section connected to said proximal portion of said distal tubular section, said lumen of said intermediate tubular section interconnecting said lumens of said proximal tubular main body and said distal tubular section, a common lumen interconnecting and extending through said proximal tubular main body, said intermediate tubular section and said distal tubular section, and wherein said intermediate tubular section is formed of a shape memory polymer having a first condition and a second condition.

2. The guide catheter of Claim 1, wherein said first condition comprises a first degree of stiffness of said intermediate tubular section, and said second condition comprises a second degree of stiffness of said intermediate tubular section, and wherein said second degree of stiffness of said intermediate tubular section is greater than said first degree of stiffness of said intermediate tubular section.

3. The guide catheter of Claim 1, wherein said first condition comprises a first degree of flexibility of said intermediate tubular section, and said second condition comprises a second degree of flexibility of said intermediate tubular section, and wherein said first degree of flexibility of said intermediate tubular section is greater than said second degree of flexibility of said intermediate tubular section.

4. The guide catheter of Claim 1, wherein said first condition comprises a first shape of said intermediate tubular section, and said second condition comprises a second shape different from said first shape of said intermediate tubular section.

5. The guide catheter of Claim 4, wherein said first shape is curved, and said second shape is substantially straight.

6. The guide catheter of any preceding Claim, wherein said shape memory polymer is in said first condition when subjected to a first predetermined temperature range, and said shape memory polymer is in said second condition when subjected to a second predetermined temperature range.

7. The guide catheter of any of Claims 1 to 5, wherein a transition of said shape memory polymer between said first condition to said second condition is induced by exposure of said shape memory polymer to a predetermined electric field.

8. The guide catheter of any of Claims 1 to 5, wherein a transition of said shape memory polymer between said first condition to said second condition is induced by exposure of said shape memory polymer to a predetermined magnetic field.

9. The guide catheter of any of Claims 1 to 5, wherein a transition of said shape memory polymer between said first condition to said second condition is induced by exposure of said shape memory polymer to a predetermined wavelength of light.

10. The guide catheter of any of Claims 1 to 5, wherein a transition of said shape memory polymer between said first condition to said second condition is induced by exposure of said shape memory polymer to a predetermined chemical solution.

11. The guide catheter of Claim 1, wherein said first condition comprises a first degree of biodegradability of said intermediate tubular section, and said second condition comprises a second degree of biodegradability of said intermediate tubular section, and wherein said intermediate tubular section is relatively more biodegradable in said second condition than in said first condition.

12. The guide catheter of Claim 4 or 5, wherein said shape memory polymer has a third condition, and wherein said third condition comprises a third shape different from said first and second shapes of said intermediate tubular section.

13. The guide catheter of Claim 2, wherein said shape memory polymer has a third condition, and wherein said third condition comprises a third degree of stiffness different from said first and second degree of stiffness of said intermediate tubular section.

14. The guide catheter of Claim 3, wherein said shape memory polymer has a third condition, and wherein said third condition comprises a third degree of flexibility different from said first and second degree of flexibility of said intermediate tubular section.

15. The guide catheter of Claim 11, wherein said shape memory polymer has a third condition, and wherein said third condition comprises a third degree of biodegradability different from said first and second degree of biodegradability of said intermediate tubular section.

16. The guide catheter of Claim 1, wherein said first and second conditions comprise first and second corresponding sets of at least one property selected from the group consisting of stiffness, flexibility, shape and biodegradability, and combinations thereof.

17. The guide catheter of Claim 16, wherein said shape memory polymer has a third condition comprising a set of at least one property corresponding to said first and second sets of at least one property of said first and second conditions, selected from the group consisting of stiffness, flexibility, shape and biodegradability, and combinations thereof.

18. The guide catheter of Claim 1, wherein a transition of said shape memory polymer between said first condition to said second condition is induced by exposure of the shape memory polymer to at least one predetermined temperature range, exposure of the shape memory polymer to at least one predetermined electric field, exposure of the shape memory polymer to at least one predetermined magnetic field, exposure of the shape memory polymer to at least one predetermined wavelength of light, or exposure of the shape memory polymer to at least one predetermined chemical solution, and combinations thereof.

19. The guide catheter of Claim 17, wherein a transition of said shape memory polymer among said first, second and third conditions is induced by exposure of the shape memory polymer to a plurality of predetermined temperature ranges, exposure of the shape memory polymer to a plurality of predetermined electric fields, exposure of the shape memory polymer to a plurality of predetermined magnetic fields, exposure of the shape memory polymer to a plurality of predetermined wavelengths of light, or exposure of the shape memory polymer to a plurality of predetermined chemical solutions, and combinations thereof.
